# EUROPEAN PATENT APPLICATION

(11) **EP 4 311 855 A1**
(43) Date of publication of application: **31.01.2024**
(21) Application number: 23188081.6
(22) Date of filing: 27.07.2023
(51) Int. Cl.: C12M 3/06, C12N 5/0793, G01N 33/50, C12M 1/00, C12M 1/12

(54) **APPARATUS AND METHOD FOR GROWING NEURAL CELLS AND COMPARTMENTALIZING AXONS AND DENDRITES**

(30) Priority: 27.07.2022 US 202263392607 P; 13.07.2023 US 202363526616 P; 21.07.2023 US 202363528245 P; 26.07.2023 US 202318226690
(71) Applicant: Xona Microfluidics, Inc., Durham, NC 27709 (US)
(72) Inventor: TAYLOR, Anne Marion, Durham, 27709 (US)
(74) Representative: Isarpatent

(57) **Abstract**

An apparatus for growing neuron cells comprises a micro-patterned microfluidic device enables fluidic isolation among microfluidic regions within the device. The device comprises first and second microfluidic regions each having an entry reservoir for accepting or extracting a first and second volume of fluid, respectively. The second volume of fluid is less than the first volume of fluid to create hydrostatic pressure. A barrier region couples the first microfluidic region with the second microfluidic region in a way that enables a biological specimen to simultaneously extend across the regions. The barrier region comprises a plurality of microgrooves having a width and height that enables the volumes of fluid to be fluidically isolated from the other via the hydrostatic pressure maintained via the at least one embedded microgroove.

## Description

### Cross-References

This application claims the benefit of U.S. Provisional Application No. 63/392,607, filed July 27, 2022, and U.S. Provisional Application No. 63/526,616, filed July 13, 2023, and U.S. Provisional Application No. 63/528,245, filed July 21, 2023, and U.S. Patent Application No. 18/226,690, filed July 26, 2023, the contents of all four of which are incorporated herein in their entirety.

### Background

An apparatus and method for growing cells is described and, more particularly, an apparatus and method for growing neuron cells using a microfluidic culture device.

Neurons represent a cell type cultured for neuroscience research, toxicity testing, and drug screening. U.S. Patent No. 7,419,822, the contents of which are hereby incorporated by reference in their entirety, describes a microfluidic neuron culture device enabling selective neuritic isolation and treatment. The microfabricated neuronal device has two or more compartments interconnected by a region having micron-sized grooves at the bottom of a physical barrier. In use, a researcher plates neuronal cells into a first somal, or cell body, compartment. The configuration of the microfluidic device enables neurites, or other portion of a cell or cellular organism, to then grow across the barrier via the grooves and extend into a second neuritic compartment while maintaining fluidic isolation. Positive or negative stimuli may then be selectively applied to distal portions of the neurites. The size of the grooves is designed to limit the neurons to the somal compartment, or chamber, while allowing the neuritic processes to grow into the neuritic compartment, thereby crossing from one compartment to another. Well-defined grooves with controlled dimension thus allow each compartment to function in a fluidically isolated manner.

Compartmentalized microfluidic neuron culturing devices have been pioneered by the Applicant, Xona Microfluidics, Inc. Such devices have become mainstays of neuron-based cell culture assays. Applicant makes and sells the microfluidic devices in two different materials: poly(dimethylsiloxane) (PDMS) and cyclic olefin copolymer (COC). The culturing device fabricated in PDMS is sold as silicone devices. Silicone devices are placed onto an optically transparent substrate (e.g., coverglass) by the end user making them optically transparent for microscopy applications. The culturing device fabricated in COC is sold under the name XonaChips^{®}. XonaChips^{®} are pre-assembled to a substrate substantially the same size as a conventional microscope slide and are optically transparent for microscopy applications.

Cells, media, and reagents are expensive and often supply-limited. It is desirable to increase sample size and the number of samples in neuron-based assays that can be processed at one time. Thus, there is a need to increase the number and density of microfluidic chambers used per area. This presents a challenge because neurons are post-mitotic, in finite supply, and require long culture times. To reduce the amount of cells used, small volumes of cell suspension and liquid could be added to such devices without introducing variability in terms of cell loading and distribution of cells.

For the foregoing reasons, there is a need to reduce the size of microfluidic devices and develop assays that use fewer neurons per culture unit so that sample size can be increased given a fixed number of neurons. In addition, a method is needed to consistently load cells in a reproducible manner. There is also a need to integrate such microfluidic chambers with more viscous matrix materials to generate 3D cultures that more closely mimic tissue found *in vivo.*

### Summary

An apparatus for growing neuron cells is provided. The neuron cell growing apparatus comprises a micro-patterned microfluidic device to direct cell attachment. The microfluidic device enables fluidic isolation among microfluidic regions within the device. The microfluidic device comprises a first microfluidic region having an entry reservoir for accepting or extracting a first volume of fluid, and a second microfluidic region having an entry reservoir for accepting or extracting a second volume of fluid that is less than the first volume of fluid to create hydrostatic pressure. A barrier region couples the first microfluidic region with the second microfluidic region in a way that enables a biological specimen to simultaneously extend across the first microfluidic region, the barrier region and the second microfluidic region. The barrier region comprises a plurality of microgrooves having a width and height that enables the second volume of fluid to be fluidically isolated from the first volume of fluid via the hydrostatic pressure maintained via the at least one embedded microgroove. The first microfluidic region, the second microfluidic region, the first entry reservoir, the second entry reservoir and the barrier region are fabricated into the device.

In one aspect, the first microfluidic region and the second microfluidic region are disposed parallel to one another and coupled with the barrier region.

In another aspect, the barrier region comprises a length of not less than 50 µm. At least one of the plurality of microgrooves comprises dimensions less than 10 µm in height.

In one feature, a biological specimen comprises a cellular structure. In one aspect, the first volume of fluid is applied to a cell body domain of the cellular structure and the second volume of fluid is applied to a cellular extension or outgrowth domain of the cellular structure. The cellular extension or outgrowth domain comprises pseudopod or lamellipodium. In another aspect, the cellular structure comprises a nerve cell.

In another feature, the first volume of fluid is applied to a somal domain of the nerve cell and the second volume of fluid is applied to an neuritic region of the nerve cell. The somal domain may comprise a nerve cell body. The neuritic region may comprise an axonal domain. The synapses of the nerve cell are isolated in the second microfluidic region.

In yet another feature, a cell-adherent coating comprises any one or more selected from the group consisting of polylysine, laminin, collagen, fibronectin, integrin, polyamine, and polyornithine.

In yet another aspect, a cross-section of a barrier in the barrier region is 7 um by 7 um.

The microgroove barrier width may be from about 75 um to about 1000 um. In one embodiment, the microgroove barrier width is about 150 um.

The first and second microfluidic regions may be about 6 mm in diameter.

The first and second entry reservoirs may be about 2 mm in diameter.

A method for growing neuron cells using a microfluidic device is also provided. The method comprises the steps of forming a micropattern configured to direct cell attachment onto a microfluidic device. A first microfluidic region is formed having a first entry reservoir for accepting or extracting a first volume of fluid. A second microfluidic region having a second entry reservoir is formed for accepting or extracting a second volume of fluid that is less than the first volume of fluid to create hydrostatic pressure. A barrier region is formed that couples the first microfluidic region with the second microfluidic region in a way that enables a biological specimen to simultaneously extend across the first microfluidic region, the barrier region and the second microfluidic region. The first volume of fluid is fluidically isolated from the second volume of fluid using the barrier region comprising a plurality of microgrooves having a width and height that enables the second volume of fluid to be fluidically isolated from the first volume of fluid via the hydrostatic pressure maintained via the at least one embedded microgroove. The first microfluidic region, the second microfluidic region, the first entry reservoir, the second entry reservoir and the barrier region are fabricated into the microfluidic device.

### Brief Description of the Drawings

For a more complete understanding of an apparatus and method for growing cells, reference should now be had to the embodiments shown in the accompanying drawings and described below. In the drawings:
FIG. 1A is schematic top plan view of an embodiment of a microfluidic device for growing neuronal cells.
FIG. 1B is a close-up of the microfluidic device taken from FIG. 1 and showing a physical barrier between compartments. The dimensions shown in correspond to the microfluidic device comprising PDMS.

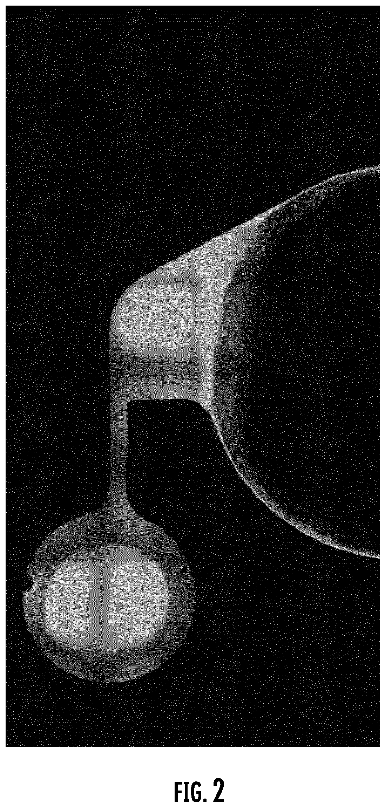

FIG. 2 is a montage of microscopic images stitched together showing a fluorescently labeled hydrogel loaded into one compartment of the PDMS microfluidic device as shown in FIG. 1.
FIG. 3 is a top plan view of a plate of a configuration of the microfluidic device made in cyclic olefin copolymer (COC) containing four culture units per chip..

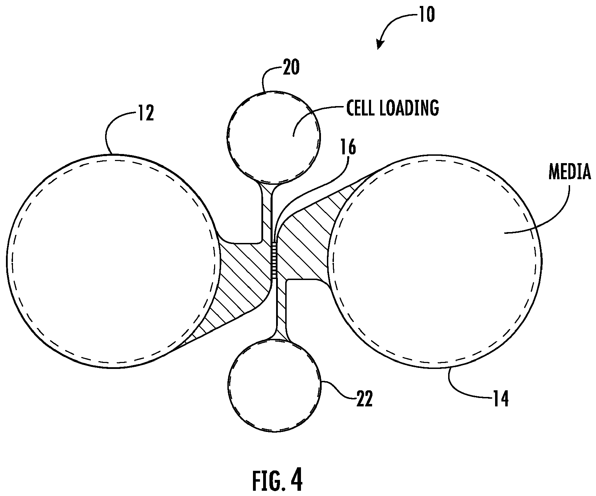

FIG. 4 is a top plan view of an embodiment of a microfluidic device for growing neuronal cells.
FIG. 5 is a top perspective view of a chip of the microfluidic device as shown in FIG. 3.
FIG. 6 is a fluorescence micrograph showing human induced pluripotent stem cell (hiPSC) differentiated into glutamatergic neurons at 7 days in culture within the microfluidic device as shown in FIG. 1.

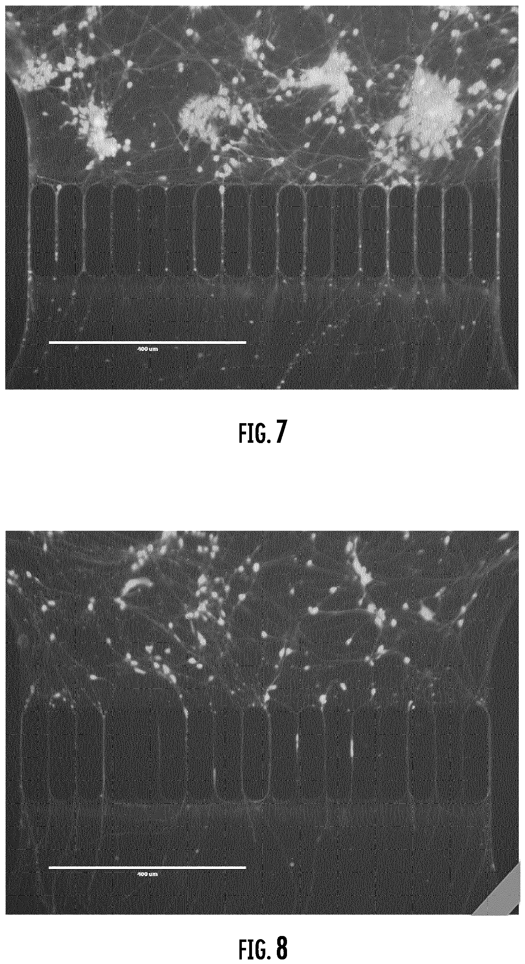

FIG. 7 is a fluorescence micrograph showing human induced pluripotent stem cells (hiPSC) differentiated into glutamatergic neurons expressing eGFP growing within the microfluidic device as shown in FIG. 1.
FIG. 8 is a fluorescence micrograph showing human induced pluripotent stem cells (hiPSC) differentiated into glutamatergic neurons expressing eGFP as shown in FIG. 7 and including hiPSC-differentiated microglia and astrocytes which are not fluorescent so they are not visible.
FIG. 9 is a stitched fluorescence micrograph image showing a larger area of hiPSC-differentiated spinal motor neurons grown for 27 days within the microfluidic device as shown in FIG. 1 and oriented clockwise 90 degrees from FIG. 8.

### Description

Certain terminology is used herein for convenience only and is not to be taken as a limiting. For example, words such as "upper," "lower," "left," "right," "horizontal," "vertical," "upward," "downward," "top" and "bottom" merely describe the configurations shown in the Figures. Indeed, the components may be oriented in any direction and the terminology, therefore, should be understood as encompassing such variations unless specified otherwise. The words "interior" and "exterior" refer to directions toward and away from, respectively, the geometric center of the core and designated parts thereof. The terminology includes the words specifically mentioned above, derivatives thereof and words of similar import.

As used herein, "axial" is deemed to mean parallel to an axis of an apparatus or device, but not necessarily coaxial therewith.

As used herein, "cell" means any cell or cells, as well as viruses or any other particles having a microscopic size, e.g. a size that is similar to that of a biological cell, and includes any prokaryotic or eukaryotic cell, e.g., bacteria, fungi, plant and animal cells. Cells are typically spherical, but can also be elongated, flattened, deformable and asymmetrical, i.e., non-spherical. The size or diameter of a cell typically ranges from about 0.1 to 120 microns, and typically is from about 1 to 50 microns. In the case of cells that are neurons, they can extend thousands of microns in length. A cell may be living or dead.

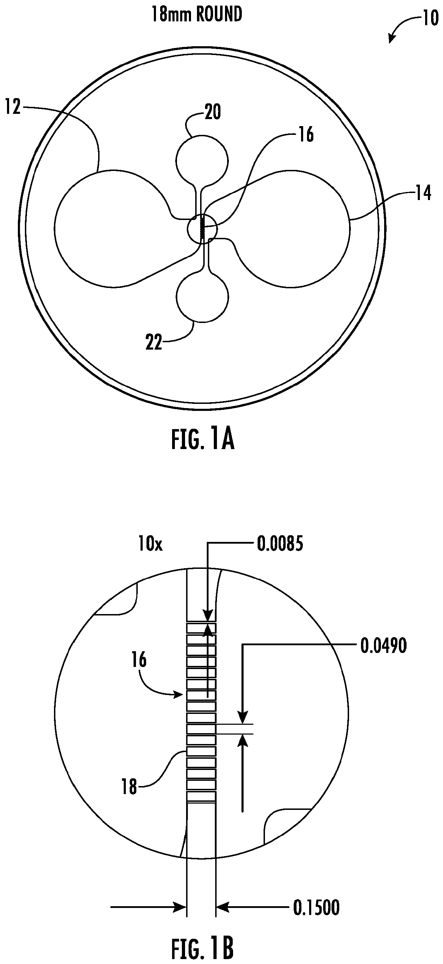

An apparatus and a method for growing cells using a microfluidic device are described. The apparatus and method may be implemented in a lab setting or in a commercial environment. Referring now to the drawings, wherein like reference numerals indicate the same or similar elements throughout the several views, an embodiment of an apparatus for growing cells is shown. As shown in FIG. 1, the apparatus comprises a microfluidic device and is generally designated at 10. The microfluidic device 10 has at least two compartments 12, 14 connected by a barrier region 16 having micron-sized grooves 18 at the bottom of a barrier region for maintaining fluidic isolation between the compartments 12, 14. The compartments are about 80 microns to about 100 microns high. The grooves are about 3 to about 8 microns high and about 75 to about 1000 microns long. The gap between each groove is about 10 to 50 microns.

The outside dimensions of the microfluidic device, or "culture unit", are the same as a standard microscope slide when embodied in cyclic olefin copolymer or a similar optically transparent injection moldable material. Each culture unit has a smaller culture surface area than in other typically used compartmentalized microfluidic devices so that fewer neurons are needed compared with previous microfluidic devices for culturing neurons. With this new culture platform, half or fewer neurons are needed compared with standard devices. The Applicant's microfluidic device is ideal for imaging-based assays with fewer microgrooves (18) than previous versions. The microgroove barrier width is 150 µm, but can easily range from 75 to 1000 µm.

The media wells are approximately 6 mm in diameter. To facilitate cell loading and reproducibility, the microfluidic device has 2 mm diameter cell loading ports 20, 22. The cell loading port 20, 22 are smaller than conventional thereby eliminating the need for angling a pipette tip during loading and makes cell loading more consistent between culture units. The small footprint device 10 reduces the number of neurons required per compartment.

The microfluidic device 10 can be applied to neuronal and non-neuronal cells. In the neuronal application, the grooves 18 are configured to guide growth of neurites and the orientation of neurite outgrowth. Fluidic isolation of the compartments within the culture area provides the ability to deliver positive or negative stimuli to one compartment to expose only localized areas of the neurons, such as the soma, axons, or dendrites.

It is understood that any number of chamber 12, 14 arrangements connected by a barrier region 16 are contemplated, including more than two independent chambers or compartments. In the embodiment shown, the microfabricated device 10 comprises a pair of separate compartmental culturing environments 12, 14, or "unit". In one embodiment, holes for the loading ports 20, 22 may be placed in each device 10. The holes 20, 22 are in fluid communication with the respective compartment 12, 14 and serve as loading inlets and cell medium reservoirs or wells for nutrient and gas exchange. For example, each unit 12, 14 of the device 10 may contain two or four holes 20, one or two at either end of each compartment 12, 14.

It is understood that various sizes, shapes or geometries, and number of grooves are possible in the microfabricated device 10. Accordingly, the microfabricated device 10 is adaptable for use in a variety of culture environments.

The microfluidic device 10 may be created using microfabrication techniques, such as photolithography, using soft lithography techniques. The microfluidic culture devices may be fabricated from poly(dimethyl siloxane), PDMS. The device may also be injection molded. The placement of the wells are designed in such a way as to facilitate visualization within the microfluidic compartments using microscopic imaging. The placement of the wells are designed to facilitate fluid loading and culture media changes. The loading ports are in alignment for all culture units when in a multi-unit configuration. The fabrication processes and materials described herein are given for purposes of example only and other methods for making the microfluidic device described are also contemplated. Both glass and plastic substrates that are flat can be used as substrates for the device. Use of an optically transparent polymer or plastic allows for live cell imaging.

The apparatus and method for growing cells has many advantages, including using fewer cells by reducing the footprint of the device as compared to the conventional device. Because of how neurons grow and mature, it is necessary to have many neurons grown together for them to functionally mature including the formation of synapses, the connections between neurons that are integral for brain development, learning and memory.. Generating human induced pluripotent stem cell derived neurons as well as other types of neurons are expensive and in finite supply because they are differentiated as post-mitotic neurons and are unable to divide and be expanded. For example, in 2023 a vial of 2.0 million human iPSC-derived motor neurons cost approximately 1,600 USD per vial (iXCells Biotechnologies). If 250,000 neurons were used for each device, only 8 samples would be able to be tested, thus it is beneficial to use fewer neurons but enough to support proper neuronal growth and development. The microfluidic compartmentalized device uses as few as 25,000 neurons, allowing 80 samples to be tested from 2.0 million cells-a 10-fold increase.

The present microfluidic device uses smaller cell loading ports to eliminate variability when loading small cell volumes, and the loading ports are aligned to facilitate automated cell loading and treatments. In addition, having a smaller microfluidic channel at the entrance near the cell loading port provides resistance to the cell suspension entering the compartment thereby reducing the flow and resulting in an even distribution of cells in the compartment. The small size of the device also increases the number of units per area.

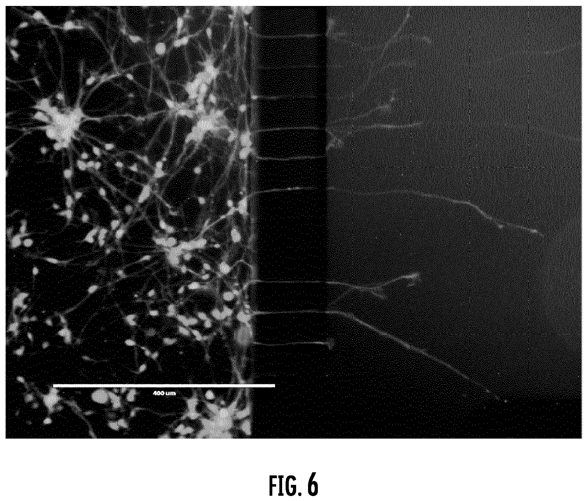

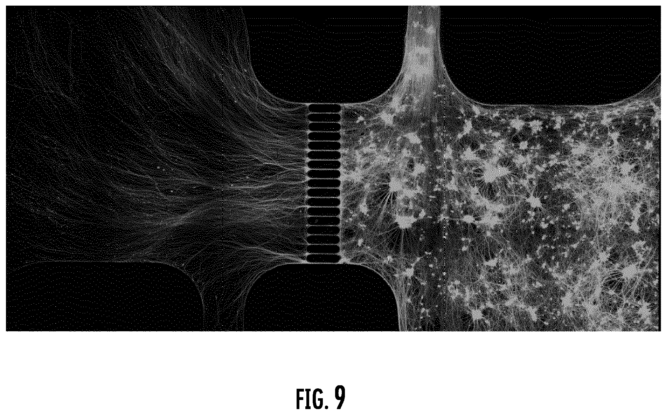

Those of ordinary skill in the art will recognize that the device described herein has applicability to other types of cells or biological type applications. In one example, FIG. 6 shows live images of the neurons which express eGFP, a fluorescent protein made by the genetically modified neurons. Expression of the eGFP shows that the neurons are alive and that they extend axons through the microgroove barrier into the axonal compartment. Similarly, FIG. 9 shows cell bodies were plated into the right compartment and axons extended into the left side. This is another live image with the motor neurons expressing eGFP.

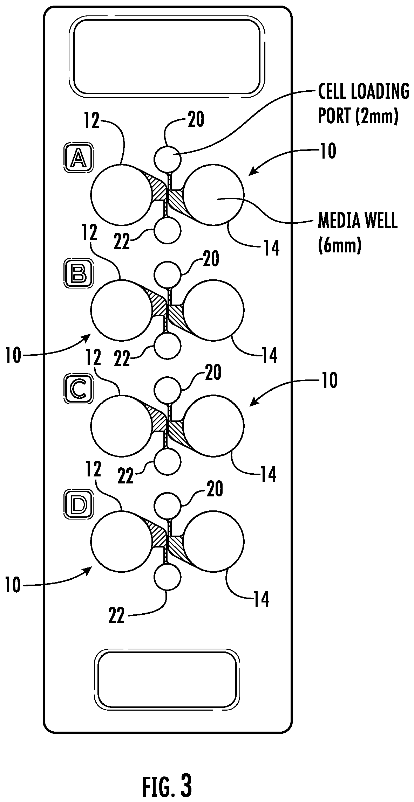

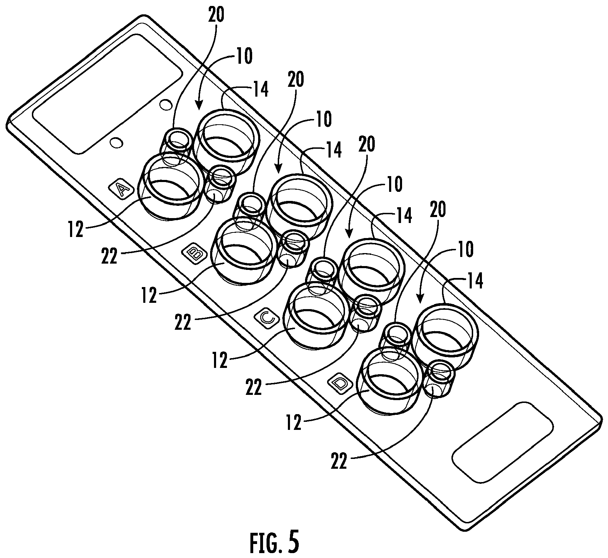

In use, a user will place the microfluidic device into a humidification tray available from Xona or other suitable sterile container such as a petri dish. 15 µl of XC Pre-Coat^{™} , which is a solvent containing a small amount of surfactant used to both sterilize the devices and prevent bubble formation, is added to the top cell loading port of culture unit A (FIGs. 3 and 5) and allowed to flow through the right compartment and into the adjoining well. This procedure is repeated for the three remaining compartmentalized culture units (B-D). The right media wells of culture units A to D are then filled with 100 µL of XC Pre-Coat^{™}. 15 µl of XC Pre-Coat^{™} are added to the lower cell loading ports of units A to D. The left media wells of unit A to D are then filled with 100 µL of XC Pre-Coat^{™} and left to sit at room temperature for 10 minutes. Solution is then aspirated form all wells, both the cell loading ports and the media wells. 15 µL of PBS is immediately added to all the upper cell loading ports. After two minutes another 15 µL of PBS is added to all the lower cell loading ports and 100 µL is added to all media wells followed by a five minute waiting period. The last two steps are repeated for a second PBS wash. The solution is aspirated from all wells. Next, 15 uL of XonaPDL, which contains poly-D-lysine, an extracellular matrix protein that help cell attachment within the device, is added to all the upper cell loading ports. After two minutes, 15 µL of XonaPDL^{™} is added to all the lower cell loading ports and then 100 µL to all media wells. The tray or petri dish is closed to prevent evaporation and placed in an incubator at 37°C for 1 h. Following incubation, place the the tray or petri dish is placed in the biosafety cabinet for 15 min to adjust room temperature. Washing steps steps 6 and7 are repeated twice and then PBS is aspirated from all wells. Cell culture media is immediately in the same manner as described in step 7. 5 µL of the neuron cell suspension prepared at 10-12 million cells per mL (∼48,000 cells in 5 µL) is loaded into the desired cell loading port(s), avoiding contact with the side walls of loading port. The microfluidic device is checked under a microscope to ensure the neurons are flowing into the main compartment. Load neurons into the remaining cell loading ports as desired for the experiment. Pause for 5 minutes for cells to attach. Approximately 15 µL of culture media is added to the cell loading port. 100 µL is added to the larger 6 mm well. The adjacent compartment is filled with 15 µL in the cell loading port and 100 µL in the larger well. The microfluidic device is returned to the incubator. After 24 h, a media change is performed by removing media from the cell loading port and media well. The channel should remain filled. 120 µL is added back to the media well. Media should be monitored every 2-3 days and changed as needed. When changing media, replace only 50% from each well and remove from the cell loading port first and then from the media well. The microfluidic device reduces the number of neurons used per compartmentalized culture unit by one half or more. In addition, the configuration provides a simpler method for loading neurons into an optimally sized loading port.

Although the apparatus and method for growing cells has been shown and described in considerable detail with respect to only a few exemplary embodiments thereof, it should be understood by those skilled in the art that the device described herein does not intend to limit the embodiments since various modifications, omissions and additions may be made to the disclosed embodiments without materially departing from the novel teachings and advantages, particularly in light of the foregoing teachings. Those of ordinary skill in the art, however, will recognize that neurons are just one example and that the device described herein has applicability to other types of cells or biological type applications. Accordingly, we intend to cover all such modifications, omission, additions and equivalents as may be included within the spirit and scope of the device as defined by the following claims. In the claims, means-plus-function clauses are intended to cover the structures described herein as performing the recited function and not only structural equivalents but also equivalent structures. Thus, although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus for growing neuron cells, the neuron cell growing apparatus comprising:
a micro-patterned microfluidic device to direct cell attachment, the device enabling fluidic isolation among microfluidic regions within the device, the microfluidic device comprising
a first microfluidic region having an entry reservoir for accepting or extracting a first volume of fluid;
a second microfluidic region having an entry reservoir for accepting or extracting a second volume of fluid that is less than the first volume of fluid to create hydrostatic pressure; and
a barrier region that couples the first microfluidic region with the second microfluidic region in a way that enables a biological specimen to simultaneously extend across the first microfluidic region, the barrier region and the second microfluidic region, the barrier region comprising
a plurality of microgrooves having a width and height that enables the second volume of fluid to be fluidically isolated from the first volume of fluid via the hydrostatic pressure maintained via the at least one embedded microgroove wherein the first microfluidic region, the second microfluidic region, the first entry reservoirs, the second entry reservoirs and the barrier region are fabricated into the device.

2. The neuron cell growing apparatus as recited in claim 1, wherein the first microfluidic region and the second microfluidic region are dispose parallel to one another and coupled with the barrier region.

3. The neuron cell growing apparatus as recited in claim 1, wherein the barrier region comprises a length of not less than 50 µm.

4. The neuron cell growing apparatus as recited in claim 1, wherein at least one of the plurality of microgrooves comprises dimensions less than 10 µm in height.

5. The neuron cell growing apparatus as recited in claim 1, wherein the biological specimen comprises a cellular structure.

6. The neuron cell growing apparatus as recited in claim 1, wherein the first volume of fluid is applied to a cell body domain of the cellular structure and the second volume of fluid is applied to a cellular extension or outgrowth domain of the cellular structure.

7. The neuron cell growing apparatus as recited in claim 1, wherein the cellular extension or outgrowth domain comprises pseudopod or lamellipodium.

8. The neuron cell growing apparatus as recited in claim 1, wherein the cellular structure comprises a nerve cell.

9. The neuron cell growing apparatus as recited in claim 1, wherein the first volume of fluid is applied to a somal domain of the nerve cell and the second volume of fluid is applied to an neuritic region of the nerve cell.

10. The neuron cell growing apparatus as recited in claim 1, wherein the somal domain comprises a nerve cell body.

11. The neuron cell growing apparatus as recited in claim 1, wherein the neuritic region comprises an axonal domain.

12. The neuron cell growing apparatus as recited in claim 1, wherein synapses of the nerve cell are isolated in the second microfluidic region.

13. The neuron cell growing apparatus as recited in claim 1, wherein the cell-adherent coating comprises any one or more selected from the group consisting of polylysine, laminin, collagen, fibronectin, integrin, polyamine, and polyornithine.

14. The neuron cell growing apparatus as recited in claim 1, wherein a cross-section of the barrier is 7 um by 7 um.

15. The neuron cell growing apparatus as recited in claim 1, wherein the microgroove barrier width is from about 75 um to about 1000 um.

16. The neuron cell growing apparatus as recited in claim 1, wherein the microgroove barrier width is about 150 um.

17. The neuron cell growing apparatus as recited in claim 1, wherein the first and second microfluidic regions are about 6 mm in diameter.

18. The neuron cell growing apparatus as recited in claim 1, wherein the first and second entry reservoirs are about 2 mm in diameter.

19. A method for growing neuron cells using a microfluidic device, the method comprising the steps of:
forming a micropattern configured to direct cell attachment onto a microfluidic device;
forming a first microfluidic region having a first entry reservoir for accepting or extracting a first volume of fluid;
forming a second microfluidic region having a second entry reservoir for accepting or extracting a second volume of fluid that is less than the first volume of fluid to create hydrostatic pressure;
forming into the microfluidic device a barrier region that couples the first microfluidic region with the second microfluidic region in a way that enables a biological specimen to simultaneously extend across the first microfluidic region, the barrier region and the second microfluidic region; and,
isolating fluidically the first volume of fluid from the second volume of fluid using the barrier region comprising a plurality of microgrooves having a width and height that enables the second volume of fluid to be fluidically isolated from the first volume of fluid via the hydrostatic pressure maintained via the at least one embedded microgroove,
wherein the first microfluidic region, the second microfluidic region, the first entry reservoir, the second entry reservoir and the barrier region are fabricated into the microfluidic device.
